# EUROPEAN PATENT APPLICATION

(11) **EP 1 917 961 A1**
(43) Date of publication of application: **07.05.2008**
(21) Application number: 06782840.0
(22) Date of filing: 14.08.2006
(51) Int. Cl.: A61K 31/23, A23L 1/30, A61K 9/14, A61K 31/231, A61K 47/48, A61P 3/04, A61P 37/04, A61P 43/00

(54) **CAPSINOID CONTAINING COMPOSITION**

(30) Priority: 16.08.2005 JP 2005235927
(71) Applicant: Ajinomoto Co., Inc., Tokyo 104-8315 (JP)
(72) Inventor: ITO, Yoko, Kawasaki-shi, Kanagawa 2108681 (JP); HIRASAWA, Kazuko, Kawasaki-shi, Kanagawa 2108681 (JP); YAMAGUCHI, Hideyuki, Kawasaki-shi, Kanagawa 2108681 (JP); MARUYAMA, Kentaro, Kawasaki-shi, Kanagawa 2108681 (JP); AMINO, Yusuke, Kawasaki-shi, Kanagawa 2108681 (JP)
(74) Representative: Strehl Schübel-Hopf & Partner
(86) International application number: PCT/JP2006/316284
(87) International publication number: WO 2007/021020

(57) **Abstract**

Problem to be Solved

To provide a technique for allowing pulverization and water-solubilization of capsinoids while maintaining their stability.

Solution

Providing a composition comprising a capsinoid compound and cyclodextrin.

## Description

### Technical Field

The present invention relates to a composition containing a capsinoid; more particularly, it relates to a new composition which is improved in preservability and useful as a raw material for anti-obesity supplement. Specifically, the invention relates to a new composition containing a complex of capsinoid and cyclodextrin.

### Background Art

It has been reported that new pungent-less capsinoid compounds (fatty acid esters of vanillyl alcohol, capsiate, dihydrocapsiate, etc., hereinafter sometimes merely referred to as "capsinoid" or "capsinoids") found in a pungent-less fixed variety of capsicum "CH-19 sweet" which was selected and fixed by Yazawa et al. as a low pungent capsicum have no pungent taste, differing from capsaicinoids (capsaicin, dihydrocapsaicin, etc.), but they exhibit an immune-activating action, activation effect for energy metabolism, and the like (JP-A-11-246478), and are expected to be put into practice in the future.

On the other hand, capsinoids exist as fat-soluble liquids at ordinary temperature, and have to be circulated in a state diluted with a highly pure liquid or oil when used as raw materials for food products. There is no problem in a food product originally blended with fats and oils, but contrivance would be required if a fat-soluble liquid capsinoid is added to and admixed with a liquid food product or powdered food product containing no fats and oils. In addition, capsinoids are very unstable because the ester linkage formed between a vanilloid group and a fatty acid chain is readily hydrolyzed. Therefore, a technique for allowing pulverization or water-solubilization of capsinoids has been required with maintenance of stability.

It is publicly known that a technique for inclusion/stabilization with cyclodextrin is effective in stabilization of a healthy functional component such as coenzyme Q-10, etc., but it is unknown that capsinoids are stabilized with cyclodextrin.

### Disclosure of Invention

The purpose of the invention is to provide a technique for allowing pulverization or water-solubilization of capsinoids applicable to a variety of preparations with maintenance of stability of capsinoids.

The present inventors assiduously worked to examine in view of the above problems and as a result found that capsinoids can be pulverized and solubilized in water in a stable state by admixing with cyclodextrin. That is, the invention includes at least the following content.
(1) A composition comprising a capsinoid compound and cyclodextrin.
(2) A composition as described in (1), wherein the capsinoid compound is capsiate, dihydrocapsiate, nordihydrocapsiate or a mixture of two or more species.
(3) A composition as described in (1) or (2), wherein cyclodextrin is one selected from the group consisting of α-cyclodextrin, β-cyclodextrin, and γ-cyclodextrin.
(4) A composition as described in any one of (1) to (3), wherein the capsinoid compound is a mixture of capsiate, dihydrocapsiate, and nordihydrocapsiate, and the cyclodextrin is γ-cyclodextrin.
(5) Acomposition as described in any one of (1) to (3), wherein the capsinoid compound is dihydrocapsiate, and the cyclodextrin is γ-cyclodextrin.
(6) Acomposition as described in any one of (1) to (5), wherein the capsinoid compound and the cyclodextrin form a complex at a molar ratio of 2 : 1 to 1 : 2.
(7) A food composition comprising a composition as described in any one of (1) to (6) and a cyclodextrin forming no complex.
(8) A process for producing a composition comprising a capsinoid compound and cyclodextrin, which comprises admixing a capsinoid compound with cyclodextrin, followed by drying and pulverizing.
(9) A process as described in (8), which comprises admixing a capsinoid compound with cyclodextrin in the molar ratio of 4 : 1 to 1 : 10.

According to the invention, a composition containing a capsinoid or capsinoids which is stably pulverized, soluble in water, easily handled and applicable to a variety of uses is provided.

### Brief Description of Drawings

Fig. 1 shows the stability of α- and γ-cyclodextrin complexes during preservation.
Fig. 2-1 shows an NMR chart of a complex consisting of a triple mixture of capsinoids and γ-cyclodextrin.
Fig. 2-2 shows an NMR chart of γ-cyclodextrin (Nihon Shokuhin Kako; Celdex G-100).

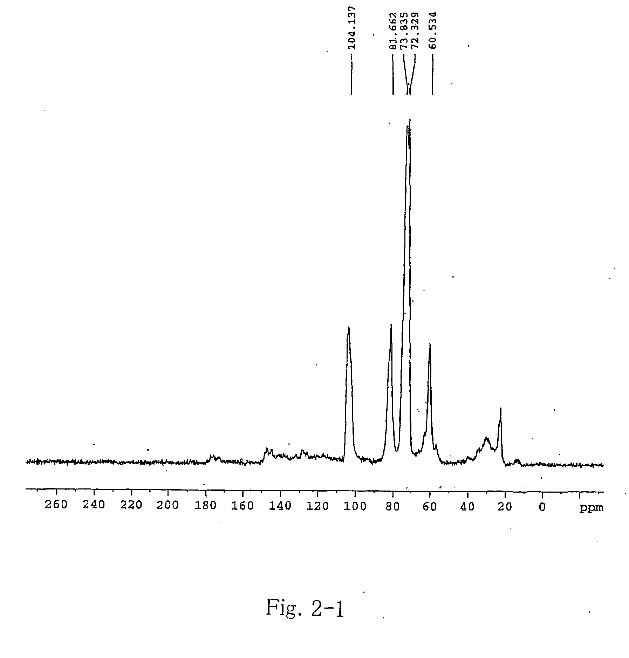

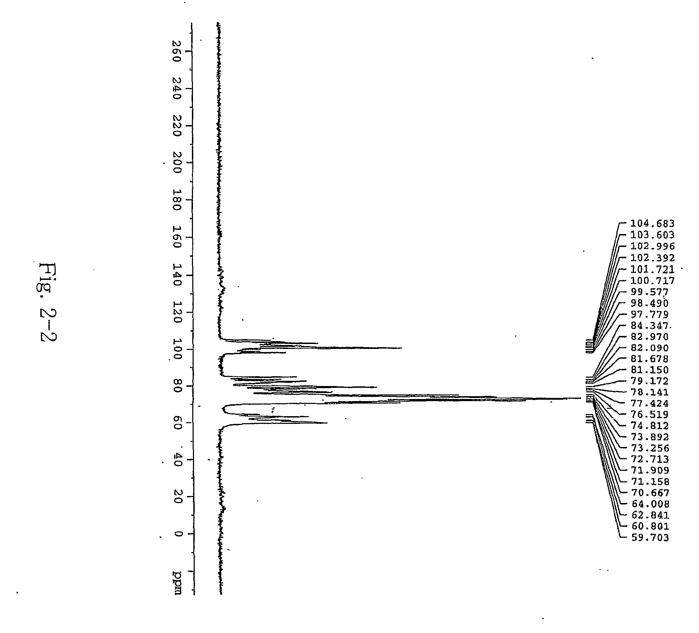

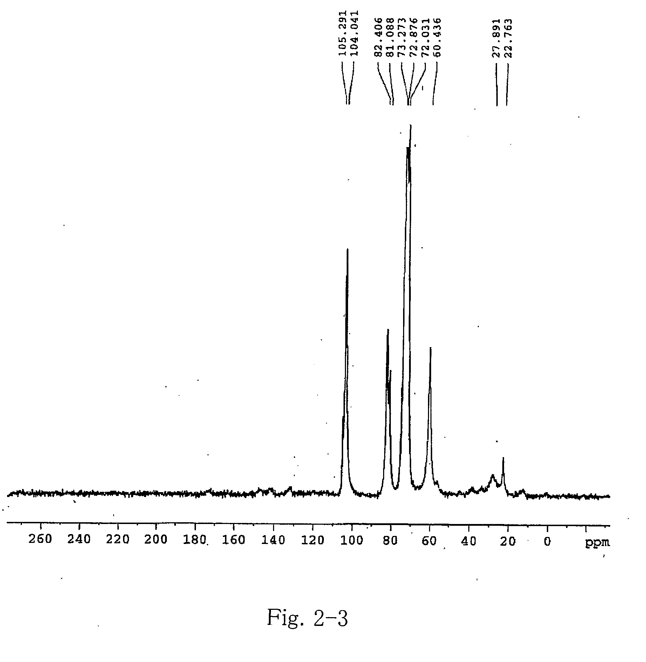

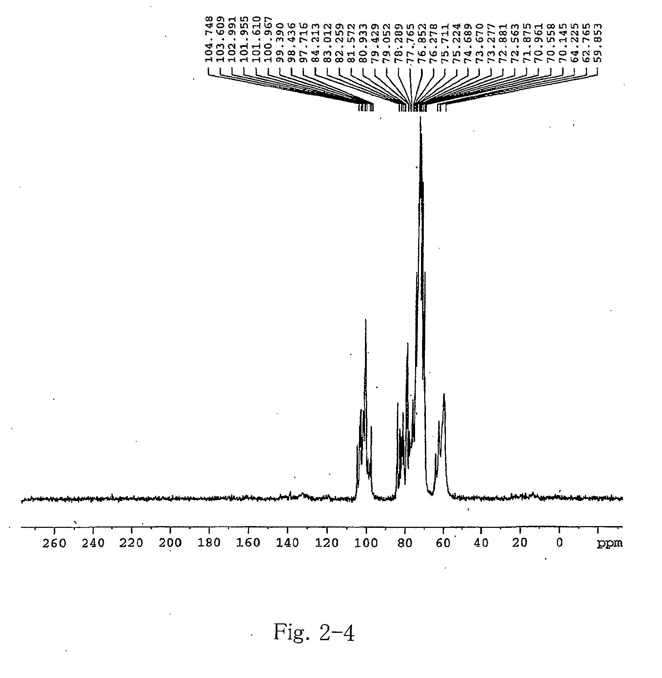

Fig. 2-3 shows an NMR chart of a complex of dihydrocapsiate and γ-cyclodextrin.
Fig. 2-4 shows an NMR chart of γ-cyclodextrin (Product of Tokyo Kasei Kogyo).

### Detailed Explanation of the Invention

### [Capsinoids]

The capsinoid compounds of the invention include fatty acid esters of vanillyl alcohol, of which the typical examples include capsiate, dihydrocapsiate, and nordihydrocapsiate confirmed as components contained in capsicum, as well as herein fatty acid esters of vanillyl alcohol with a variety of straight chain or branched chain fatty acids having approximately the same chain length as that of capsiate or nordihydrocapsiate, such as vanillyl decanoate, vanillyl nonanoate, vanillyl octanoate, and the like. Capsiate (hereinafter, sometimes abbreviated to CST), dihydrocapsiate (hereinafter, sometimes abbreviated to DCT) and nordihydrocapsiate (hereinafter, sometimes abbreviated to NDCT), respectively can be represented by the following chemical formulae.

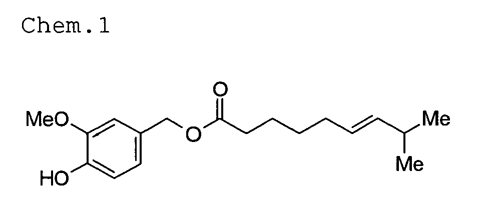

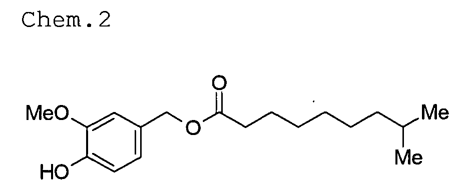

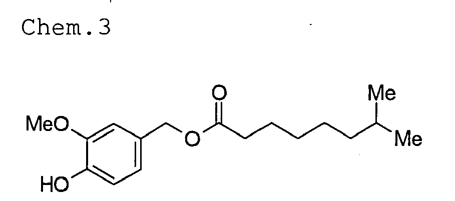

Capsinoids which are abundant in plants belonging to Capsicum can be prepared by purifying and isolating from the plant body and/or seeds of the plant belonging to Capsicum. The capsicum used in purification may be an ordinary species of Capsicum represented by "Nikko", "Goshiki", and the like, and any species of Capsicum may be used as far as it contains capsinoids. In particular, pungent-less ordinary species of Capsicum represented by "CH-19 sweet", "Manganji", "Fusimi Kancho", green pepper, sweet pepper, and the like can be employed preferably since they contain a large quantity of capsinoids. In addition, it is particularly preferable to use a pungent-less species "CH-19 sweet" since it contains said component in large quantities. Herein, the term "CH-19 sweet" means "CH-19 sweet" per se and progeny relative species derived from "CH-19 sweet", and in this specification the term "CH-19 sweet" is used to include all of these species. Purification and isolation may be achieved in a conventional manner well known by a person skilled in the art, for example, extraction with a solvent, various types of chromatography such as silica gel chromatography, preparative high performance liquid chromatography, and the like, alone or in appropriate combination; for example, a method as described in JP-A-11-246478 may be employed.

Further, the above-mentioned capsinoids may be synthesized by an ester exchange reaction of a corresponding fatty acid ester with vanillyl alcohol as a starting material as described in JP-A-11-246478. Alternatively, capsinoids may be synthesized in the other manner well known by a person skilled in the art based on their structure. Further, capsinoids may easily be prepared in a synthetic method using an enzyme. For example, the desired capsinoid compounds may be prepared by utilizing a reverse reaction of lipase from vanillyl alcohol and a compound such as a fatty acid ester and/or triglyceride corresponding to the desired compound as substrates in a method as described in JP-A-2000/312598 or Kobata et al., Biosci. Biotechnol. Biochem., 66(2), 319-327, 2002).

The capsinoid compound, when used in preparation of the composition of the invention, may be an extract as mentioned above or a synthetic product, and may be used alone or as a mixture of two or more compounds. Additionally, the capsinoid compounds may contain their degradation products such as free fatty acid or vanillyl alcohol. The capsinoid compound, when it is an extract from Capsicum, can be used in preparation of the composition of the invention in a state of a mixture with fats and oils. Accordingly, this process for preparation may be applied to the capsinoid compound containing naturally occurring fats and oils or the capsinoid compound dissolved once in fats and oils after extraction in order to separate and purify the capsinoid compound from fats and oils. When an extract product is used, it is a mixture of the above three compounds as main components, which approximate each other in their molecular size and can be used in preparation of the composition without any problem.

### [Cyclodextrin]

Cyclodextrin means cyclic oligosaccharides in which 6 to 12 pieces of D-glucose form a ring structure through α1 → 4 linkage, which are prepared from starch by conversion with an enzyme such as CGTase (cyclodextrin glucanotransferase, CD generating enzyme).

The cyclodextrin (hereinafter sometimes referred to as "CD") used in the invention at low cost includes non-branched cyclodextrins such as α-cyclodextrin (polymerization degree: 6), β-cyclodextrin (polymerization degree: 7), γ-cyclodextrin (polymerization degree: 8), and additionally chemically modified cyclodextrins (branched cyclodextrin) in which non-branched cyclodextrin molecule is methylated, hydroxypropylated, mono-acetylated, tri-acetylated, mono-chlorotriazino-, sulfobutylated, or glycosyl-maltosylated, can be used. The invention is not limited by these cyclodextrins.

In this invention, the cyclodextrin may be used alone or as a mixture, though γ-cyclodextrin is particularly preferred.

### [Process for Preparing a Composition Containing Capsinoid]

The composition of the invention containing capsinoid, that is, the composition containing a capsinoid compound and cyclodextrin, can be prepared in a variety of publicly known processes such as a saturated solution process or kneading process. In the saturated solution process, a saturated solution of cyclodextrin is prepared, to which a capsinoid compound is added; after mixing, an inclusion complex is deposited from the mixture, collected as crystals, which are dried to yield powder. In the kneading process, water is added to cyclodextrin to yield a slurry mixture, to which a capsinoid compound is added and kneaded, and dried to yield powder of the complex (Nippon Shokuhin Kagaku Kogaku Kaishi, vol.51, no.12, 647-655 (2004); general review). Drying may be selected properly from freeze-drying, spray drying, drying under reduced pressure, drying under hot air, and the like.

In this operation, the molar ratio of a capsinoid compound to cyclodextrin in the mixture is approximately 4 : 1 to 1 : 10, preferably about 2 : 1 to 1 : 2. The resulting mixture prepared in this ratio forms a complex comprising the capsinoid compound and cyclodextrin in the ratio of about 2 : 1 to 1 : 2. Thus, the composition of the invention can be characterized in view of either the mixing ratio in the preparation or the ratio of formation for the complex.

As the solvent used for dissolving cyclodextrin during mixture formation, water or a variety of buffer solutions such as citrate buffer may properly be employed, and preferably water is used in view of convenience for the preparation. On the other hand, capsinoid is in an oil form at ordinary temperature, and in using it may be used as such or diluted in an organic solvent such as ethanol. In forming a complex with α-cyclodextrin, it is preferable to avoid mixing of the capsinoid with an organic solvent.

When the composition obtained by admixing is obtained as precipitates in the solution, after filtration, it may be dried as such in a state of mixture with free cyclodextrin not forming the complex; the complex before drying may be washed with an organic solvent such as hexane, pentane, heptane, alcohol, a mixture of alcohol and water, etc., and then dried. The capsinoid which has not formed the complex is eliminated by washing with an organic solvent; this operation is effective in improvement of stickiness.

The composition of the invention prepared as mentioned above is in a state of powder at ordinary temperature, which has been pulverized from the capsinoid compound so far being in an oily state; thus, this allows preparation of a variety of formulations which have not so far been attained in the oily state. The composition of the invention can be formed in any mold different in form, which may be prepared according to the purpose of use. In addition, the composition of the invention can be blended in a conventional composition for a variety of supplements, for example, supplement forms such as granules, tablets, hard capsules; food forms such as drinks, powdered drinks, instant soup, chocolate, candies, chewing gum, tablet confection, bakery confection, snacks, processed marine products, stock farm meat processed products, retort foods, frozen foods, instant noodle, health foods, etc.; thus, the composition is very useful since it allows easy ingestion of the capsinoids which are also diet components.

### Examples

### Example 1

### Trial Preparation of the Composition Using γ-Cyclodestrin

γ-Cyclodextrin (Nihon Shokuhin Kako; trade name: Celdex G-100) (18 . 6 g; 14.3 mmol) was dissolved in 74 g of 0.1 mM citrate buffer (pH 3.5) to yield a saturated solution of γ-cyclodextrin. As capsinoids, a triple mixture of capsinoids (4.39 g; 14.3 mmol) (synthesized according to the method as described by Kobata et al., Biosci. Biotechnol. Biochem., 66(2), 319-327, 2002; triple mixture (88% purity) containing capsiate, nordihydrocapsiate, and dihydrocapsiate at the ratio of 62 : 7 : 30) was used. The synthesized capsinoid was dissolved in 6 mL of ethanol in advance. The saturated solution of γ-cyclodextrin was powerfully agitated with a homo-mixer (Heidolph, DIAX900) at dial 2 (corresponding to 11,000 rpm), to which was added capsinoid. The initially clear solution became cloud immediately after dropwise addition; occurrence of precipitates was confirmed visually. After stirring for 10 minutes, the mixture was centrifuged (8, 000 rpm, 10 minutes) to recover the precipitates, which were freeze-dried to yield 18.1 g of complex powder of capsinoid and γ-cyclodextrin. The resulting γ-cyclodextrin complex powder was dissolved in DMF (N,N-dimethylformamide), diluted with a mixture of ethyl acetate and methanol (6 : 4), and analyzed by high performance liquid chromatography (HPLC). The result of HPLC analysis indicated the capsinoid content being 23.2% as the total amount of three species of capsinoids.

### Example 2

### Trial Preparation of the Composition Using α-Cyclodestrin and Examination of Stability during Preservation

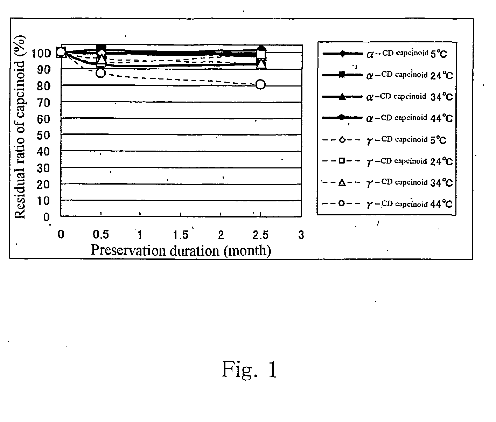

α-Cyclodextrin (Nihon Shokuhin Kako; trade name: Celdex A-100) (8.1 g; 8.3 mmol) and triple mixture of capsinoids (2.54 g; 8.3 mmol) were used; otherwise in the same manner as in Example 1, the complex powder (4.9 g) of α-cyclodextrin and capsinoid (capsinoid content: 45.9%) was obtained. On the resulting complex powder and the complex powder of γ-cyclodextrin (capsinoid content: 29.4%) prepared in Example 1, the stability during preservation was evaluated, respectively. Fig. 1 shows the results. The complex powder (100 mg) was placed in an aluminum pouch, and preserved at a temperature of 5°C, 24°C, 34°C or 44°C and a relative humidity of 78%RH. In the cyclodextrin complex powders, high stability of capsinoid was confirmed.

### Examples

### Example 3

### Trial Preparation of the Composition Using β-Cyclodestrin and Examination of Solubility in Water

β-Cyclodextrin (Nihon Shokuhin Kako; trade name: Celdex B-100) (1.28 g; 1.13 mmol) and a triple mixture of capsinoids (0.346 g; 1.13 mmol) were used; otherwise in the same manner as in Example 1, the complex powder (0.769 g) of β-cyclodextrin and capsinoids (capsinoid content: 33.7%) was obtained. On the respective complex powder of α, β, or γ-cyclodextrin and capsinoids prepared in Examples 1 to 3, the solubility in water was evaluated. Table 1 shows the results. The complex powder was slowly added to 100 mL of buffer (pH 3), and the dissolved amount and the floating oil occurring during dissolution were evaluated visually. Though there is some difference in the amount dissolved in water depending on the kind of cyclodextrin, the water-solubility of the complex powder with capsinoid was confirmed.

**Table 1. Solubility of cyclodextrin complexes in water**

| Sample | Dissolved amount | Floating oil |
|---|---|---|
| α-Cyclodextrin complex powder (Example 2) | Capsinoid corresp. to ca 10 mg/100 mL | trace |
| β-Cyclodextrin complex powder (Example 3) | - (unable to calc.) | large quantity |
| γ-Cyclodextrin complex powder (Example 1) | Capsinoid corresp. to ca 0.3 mg/100 mL | none |

### Example 4

In the same manner as in Example 1, on 3 types of α, β, and γ-cyclodextrins, the 4 types of complexes of capsinoid : cyclodextrin = 3 : 1, 2 : 1, 1 : 1, and 1 : 2 by molar ratio were prepared on trial. In reference to the value as described in the published document, cyclodextrin each was dissolved in 80 mL of water up to saturation, and then capsinoid was added depending on the fixed amount of cyclodextrin so that the molar ratio of blended capsinoid became the values as shown in Table. Table 2 shows the list of trial blending and the result of the complexes produced (recovery of capsinoid, stickiness of the complex powder). In all of the blending, preparation of the complex powder was allowed, but as powder the mixture with γ-cyclodextrin was preferred because of less stickiness. In particular, the use of an equimolar or more amount of γ-cyclodextrin for capsinoid in the mixture was preferred.

**Table 2: Trial blending in the complexes and trial results**

| Cyclodextrin (CD) Type | CD:Capsinoid Molar ratio of blending | Capsinoid Recovery (%) | Stickiness in Complex Powder* |
|---|---|---|---|
| α-CD | 3:1 | 85.6 | + |
| | 2:1 | 80.0 | + |
| | 1:1 | 90.1 | + |
| | 1:2 | 92.8 | ++ |
| β-CD | 3:1 | 57.9 | + |
| | 2:1 | 66.5 | + |
| | 1:1 | 75.0 | + |
| | 1:2 | 81.7 | ++ |
| γ-CD | 3:1 | 85.2 | - |
| | 2:1 | 90.7 | - |
| | 1:1 | 95.8 | - |
| | 1:2 | 99.5 | ++ |

| | | | |
|---|---|---|---|
| *-: No stickiness; +:slight stickiness; ++ strong stickiness | | | |

### Example 5

### Formation of a complex of γ-cyclodextrin and dihydrocapsiate

(1) γ-Cyclodextrin (649.1 mg) (Tokyo Kasei Kogyo Co., Ltd.; 0.5 mmol) was dissolved in 2 ml of water at 40°C and cooled to room temperature to yield a saturated aqueous solution of cyclodextrin. Dihydrocapsiate (154.6 mg, 0.5 mmol; containing 0.08 equivalent of 8-methylnonanoic acid) was dissolved in 0.5 ml of ethanol at room temperature and added to the saturated aqueous solution of cyclodextrin. The solution was stirred at room temperature for 2 hours, and the precipitated crystals were collected by filtration. The crystals were washed with 5 ml of hexane and dried under reduced pressure to yield 584.1 mg of dihydrocapsiate/γ-cyclodextrin complex as white powder. According to ¹H-NMR measurement (solvent= DMSO-d6 (deuterated dimethylsulfoxide), measured with an AVANCE400, Bruker Co., Ltd., the same hereafter), this complex comprised DCT : γ-CD = 1 : 0.88 by molar ratio, and contained 0.10 equivalent by mole of 8-methylnonanoic acid for DCT.
(2) γ-Cyclodextrin (937.1 mg) (Tokyo Kasei Kogyo Co., Ltd.; 0.75 mmol) was dissolved in 3 ml of water to yield a saturated aqueous solution of cyclodextrin; otherwise in the same manner as in (1), 610.4 mg of dihydrocapsiate/γ-cyclodextrin complex was obtained as white powder. According to ¹H-NMR measurement (solvent= DMSO-d6), this complex comprised DCT : γ-CD = 1 : 1 by molar ratio, and contained 0.08 equivalent by mole of 8-methylnonanoic acid for DCT.
(3) Dhydrocapsiate (308.6 mg) (Tokyo Kasei Kogyo Co., Ltd.; 0.75 mmol) was used; otherwise in the same manner as in (1), 697.9 mg of dihydrocapsiate/γ-cyclodextrin complex was obtained as white powder. According to ¹H-NMR measurement (solvent= DMSO-d6), this complex comprised DCT : γ-CD = 1 : 1 by molar ratio, and contained 0.15 equivalent by mole of 8-methylnonanoic acid for DCT.
(4) γ-Cyclodextrin (648.3 mg) (Tokyo Kasei Kogyo Co., Ltd.; 0.5 mmol) was dissolved in 3 ml of citrate buffer (pH 3.5) at 40°C and then cooled to room temperature to yield a saturated solution of cyclodextrin. Dihydrocapsiate (154.2 mg, 0.5 mmol) was dissolved in 0.5 ml of ethanol at room temperature and added to the saturated solution of cyclodextrin. The solution was stirred at room temperature for 2 hours, and the precipitated crystals were collected by filtration. The crystals were washed with 5 ml of hexane and dried under reduced pressure to yield 733.0 mg of dihydrocapsiate/γ-cyclodextrin complex as white powder. According to ¹H-NMR measurement (solvent= DMSO-d6), this complex comprised DCT : γ-CD = 1 : 1 by molar ratio, and contained 0.09 equivalent by mole of 8-methylnonanoic acid for DCT.
(5) To a saturated solution of cyclodextrin was added a dihydrocapsiate/ethanol solution, and the mixture was stirred for 15 minutes, and otherwise treated in the same manner as in (4) to yield 584.0 mg of dihydrocapsiate/γ-cyclodextrin complex as white powder. According to ¹H-NMR measurement (solvent= DMSO-d6), this complex comprised DCT : γ-CD = 1 : 0.8 by molar ratio, and contained 0.10 equivalent by mole of 8-methylnonanoic acid for DCT.

Table 3 shows the results of (1) to (5). Thus, in any case of 2 : 1 to 1 : 2 in the mixing ratio of the raw materials, DCT : γ-CD, a good complex was formed. As for the solvent for cyclodextrin, both of water and a citrate buffer could be used satisfactorily. Additionally, it was confirmed that the complex was formed in the ratio of approximately 1 : 1 for DCT : γ-CD.

**Table 3. Formation of the complex of γ-cyclodextrin and dihydrocapsiate**

| Exp. | Raw Mat. | | Solvent | | | Stirring | Yield | Recovery | | Incl. cmp. (for DCT) | |
|---|---|---|---|---|---|---|---|---|---|---|---|
| | CD | DCT | CD | | DCT (EtOH) | | | Incl cmp^{*1} | DCT^{*2} | CD con. | Side chain con^{*3} |
| | mmol | mmol | solv | ml | ml | hr | mg | % | % | eq | eq |
| 5-1 | 0.5 | 0.5 | H₂O | 2 | 0.5 | 2 | 584.1 | 72.7 | 80.4 | 0.9 | 0.10 |
| 5-2 | 0.75 | 0.5 | H₂O | 3 | 0.5 | 2 | 610.4 | 54.1 | 76.8 | 1.0 | 0.08 |
| 5-3 | 0.5 | 1.0 | H₂O | 2 | 0.5 | 2 | 697.9 | 72.9 | 44.5 | 1.0 | 0.15 |
| 5-4 | 0.5 | 0.5 | CA* | 3 | 0.5 | 2 | 733.0 | 91.3 | 91.3 | 1.0 | 0.10 |
| 5-5 | 0.5 | 0.5 | CA* | 3 | 0.5 | 0.25 | 584.0 | 72.8 | 86.8 | 0.8 | 0.10 |

| | | | | | | | | | | | |
|---|---|---|---|---|---|---|---|---|---|---|---|
| *: aq. citric acid *1: [inclusion cmp. by wt/(CD+DCT by wt)] x 100 *2: [DCT in inclusion cmp. by wt/DCT used] x 100 *3: 8-methylnonanoic acid | | | | | | | | | | | |

### Example 6

### Formation of the complexes of γ-cyclodextrin and a variety of capsinoids

(1) γ-Cyclodextrin (648.7 mg) (Tokyo Kasei Kogyo Co., Ltd.; 0.5 mmol) was dissolved in 2 ml of water at 40°C and then cooled to room temperature to yield a saturated aqueous solution of cyclodextrin. Capsiate (154.0 mg, 0.5 mmol, containing 0.63 eq. of trans-8-methyl-6-nonenoic acid) was dissolved in 0.5 ml of ethanol at room temperature and added to the saturated aqueous solution of cyclodextrin. The solution was stirred at room temperature for 2 hours, and the precipitated crystals were collected by filtration. The crystals were washed with 5 ml of hexane and dried under reduced pressure to yield 648.2 mg of capsiate/γ-cyclodextrin complex as white powder. According to ¹H-NMR measurement (solvent= DMSO-d6), this complex comprised CST : γ-CD = 1 : 1 by molar ratio, and contained 0.61 equivalent by mole of trans-8-methyl-6-nonenoic acid for CST.
(2) In place of capsiate, nordihydrocapsiate (147.6 mg; 0.5 mmol, containing 0.08 equivalent of 7-methyloctanoic acid; the same hereinafter) was used; otherwise, in the same manner as in (1), 511.0 mg of nordihydrocapsiate/γ-cyclodextrin complex was obtained as white powder. According to ¹H-NMR measurement (solvent= DMSO-d6), this complex comprised NDCT : γ-CD = 1 : 0.8 by molar ratio, and contained 0.09 equivalent by mole of 7-methyloctanoic acid for NDCT.
(3) In place of capsiate, vanillyl decanoate (hereinafter, sometimes abbreviated to as VD) (155.2 mg; 0.5 mmol, containing 0.08 equivalent of n-decanoic acid, the same hereinafter) was used; in the same manner as in (1), 425.3 mg of vanillyl decanoate/ γ-cyclodextrin complex was obtained as white powder. According to ¹H-NMR measurement (solvent= DMSO-d6), this complex comprised VD : γ-CD = 1 : 1 by molar ratio, and contained 0.09 equivalent by mole of n-decanoic acid for VD.

Table 4 shows the results of (1) - (3) . Thus, the results confirmed that in any case of examination using a variety of capsinoids a good complex with cyclodextrin was formed.

**Table 4: Formation of the complex of capsinoid and γ-cyclodextrin**

| Exmp. | Capsinoid | Yield | Recovery | | Inclusion cmp. (for capsinoid) | |
|---|---|---|---|---|---|---|
| | | | Inclusion cmp^{*1} | Capsinoid ^{*2} | CD content | Side chain con^{*3} |
| | | [mg] | [%] | [%] | [eq] | [eq] |
| 6-1 | Capsiate | 648.2 | 80.8 | 83.8 | 1.0 | 0.61 |
| 6-2 | Nordihydrocapsiate | 511.0 | 64.2 | 74.3 | 0.8 | 0.09 |
| 6-3 | Vanillyl decanoate | 425.3 | 52.9 | 51.8 | 1.0 | 0.09 |

| | | | | | | |
|---|---|---|---|---|---|---|
| *1:[inclusion cmp. by wt/(CD+capsinoid by wt)] x 100 *2:[capsinoid in inclusion cmp. by wt/capsinoid used] x 100 *3: No.6: trans-8-methyl-nonenoic acid; No.7: 7-methylocatanoic acid; No.8: n-decanoic acid | | | | | | |

### Example 7

### Formation of the complex of β-cyclodextrin and dihydrocapsiate

(1) β-Cyclodextrin (567.8 mg) (Tokyo Kasei Kogyo Co., Ltd.; 0.5 mmol) was dissolved in 15 ml of water at 40°C and then cooled to room temperature to yield a saturated aqueous solution of cyclodextrin. Dihydrocapsiate (155.7 mg, 0.5 mmol) was dissolved in 0.5 ml of ethanol at room temperature and added to the saturated aqueous solution of cyclodextrin. The solution was stirred at room temperature for 2 hours, and the precipitated crystals were collected by filtration. The crystals were washed with 5 ml of hexane and dried under reduced pressure to yield 580.2 mg of dihydrocapsiate/β-cyclodextrin complex as white powder. According to ¹H-NMR measurement (solvent= DMSO-d6), this complex comprised DCT : β-CD = 1 : 1.3 by molar ratio, and contained 0.12 equivalent by mole of 8-methylnonanoic acid for DCT.
(2) β-Cyclodextrin (1.135 g) (Tokyo Kasei Kogyo Co., Ltd.; 1.0 mmol) was dissolved in 30 ml of water to yield a saturated aqueous solution of cyclodextrin; otherwise, this was treated in the same manner as in (1) to yield 865.7 mg of dihydrocapsiate/β-cyclodextrin complex as white powder. According to ¹H-NMR measurement (solvent= DMSO-d6), this complex comprised DCT : β-CD = 1 : 2 by molar ratio, and contained 0.09 equivalent by mole of 8-methylnonanoic acid for DCT.
(3) Dihydrocapsiate (231.9 mg; 0.75 mmol) was used; otherwise, in the same manner as in (1), 586.0 mg of dihydrocapsiate/β-cyclodextrin complex was obtained as white powder. According to ¹H-NMR measurement (solvent= DMSO-d6), this complex comprised DCT : β-CD = 1 : 1.8 by molar ratio, and contained 0. 16 equivalent by mole of 8-methylnonanoic acid for DCT.
(4) β-Cyclodextrin (567.5 mg) (Tokyo Kasei Kogyo Co., Ltd.; 0.5 mmol) was dissolved in 15 ml of citrate buffer (pH 3.5) at 40°C and then cooled to room temperature to yield a saturated solution of cyclodextrin. Dihydrocapsiate (154.2 mg, 0.5 mmol) was dissolved in 0.5 ml of ethanol at room temperature and added to the saturated solution of cyclodextrin. The solution was stirred at room temperature for 2 hours, and the precipitated crystals were collected by filtration. The crystals were washed with 5 ml of hexane and dried under reduced pressure to yield 463.8 mg of dihydrocapsiate/β-cyclodextrin complex as white powder. According to ¹H-NMR measurement (solvent= DMSO-d6), this complex comprised DCT : β-CD = 1 : 1.5 by molar ratio, and contained 0.09 equivalent by mole of 8-methylnonanoic acid for DCT.
(5) To a saturated cyclodextrin solution was added a dihydrocapsiate/ethanol solution, and the mixture was stirred for 15 minutes, and otherwise treated in the same manner as in (4) to yield 318.7 mg of dihydrocapsiate/β-cyclodextrin complex as white powder. According to ¹H-NMR measurement (solvent= DMSO-d6), this complex comprised DCT : β-CD = 1 : 0.8 by molar ratio, and contained 0.11 equivalent by mole of 8-methylnonanoic acid for DCT.

Table 5 shows the results of (1) to (5). Thus, in any case of 2 : 1 to 1 : 2 in the mixing ratio of the raw materials, DCT : β-CD, a good complex could be formed. As for the solvent for cyclodextrin, both of water and a citrate buffer could be used satisfactorily. Additionally, it was confirmed that the complex was formed in the ratio of approximately 1 : 1 to 1 : 2 for DCT : β-CD.

**Table 5: Formation of the complex of dihydrocapsiate with β-cyclodextrin**

| Exp. | Raw Mat. | | Solvent | | Stirring | Yield | Recovery | | Incl. cmp. (for DCT) | | |
|---|---|---|---|---|---|---|---|---|---|---|---|
| | CD | DCT | CD | | DCT (EtOH) | | | Incl mp^{*1} | DCT^{*2} | CD con. | Side chain con^{*3} |
| | mmol | mmol | solv | ml | ml | hr | mg | % | % | eq | eq |
| 7-1 | 0.5 | 0.5 | H₂O | 15 | 0.5 | 2 | 580.2 | 80.2 | 63.2 | 1.3 | 0.12 |
| 7-2 | 1.0 | 0.5 | H₂O | 30 | 0.5 | 2 | 865.7 | 67.1 | 67.1 | 2.0 | 0.09 |
| 7-3 | 0.5 | 0.75 | H₂O | 15 | 0.5 | 2 | 586.0 | 73.3 | 32.8 | 1.8 | 0.16 |
| 7-4 | 0.5 | 0.5 | CA* | 15 | 0.5 | 2 | 463.8 | 64.2 | 46.1 | 1.5 | 0.09 |
| 7-5 | 0.5 | 0.5 | CA* | 15 | 0.5 | 0.25 | 318.7 | 44.2 | 52.4 | 0.8 | 0.11 |

| | | | | | | | | | | | |
|---|---|---|---|---|---|---|---|---|---|---|---|
| *: aq. citric acid *1:[inclusion cmp. by wt/(CD+DCT by wt)] x 100 *2:[DCT in inclusion cmp. by wt/DCT used] x 100 *3: 8-methylnonanoic acid | | | | | | | | | | | |

### Example 8

### Formation of the complex of vanillyl decanoate with β-cyclodextrin

β-Cyclodextrin (568.0 mg) (Tokyo Kasei Kogyo Co., Ltd.; 0.5 mmol) was dissolved in 15 ml of water at 40°C and then cooled to room temperature to yield a saturated aqueous solution of cyclodextrin. Vanillyl decanoate (154.3 mg, 0.5 mmol) was dissolved in 0.5 ml of ethanol at room temperature and added to the saturated aqueous solution of cyclodextrin. The solution was stirred at room temperature for 2 hours, and the precipitated crystals were collected by filtration. The crystals were washed with 5 ml of hexane and dried under reduced pressure to yield 554.2 mg of vanillyl decanoate/β-cyclodextrin complex as white powder. According to ¹H-NMR measurement (solvent= DMSO-d6), this complex comprised VD : β-CD =1 : 1.9 by molar ratio, and contained 0.12 equivalent by mole of n-decanoic acid for VD.

**Table 6: Formation of the complex of vanillyl decanoate and β-cyclodextrin**

| Exmp. | Capsinoid | Yield | Recovery | | Inclusion cmp. (for VD) | |
|---|---|---|---|---|---|---|
| | | | Inclusion cmp*¹ | VD^{*2} | CD content | Side chain con^{*3} |
| | | [mg] | [%] | [%] | [eq] | [eq] |
| 8 | vanillyl decanoate | 554.2 | 76.7 | 44.9 | 1.9 | 0.12 |

| | | | | | | |
|---|---|---|---|---|---|---|
| *1:[inclusion cmp. by wt/(CD+VD by wt)] x 100 *2:[VD in inclusion cmp. by wt/VD used] x 100 *3: n-decanoic acid | | | | | | |

### Example 9

### Formation of the complex of dihydrocapsiate with α-cyclodextrin

(1) α-Cyclodextrin (486.4 mg) (Tokyo Kasei Kogyo Co., Ltd.; 0.5 mmol) was dissolved in 2 ml of water at 40°C and then cooled to room temperature to yield a saturated aqueous solution of cyclodextrin. Dihydrocapsiate (154.2 mg, 0.5 mmol) was dissolved in 0.5 ml of ethanol at room temperature and added to the saturated aqueous solution of cyclodextrin. The solution was stirred at room temperature for 2 hours, and the precipitated crystals were collected by filtration. The crystals were washed with 5 ml of hexane and dried under reduced pressure to yield 297.5 mg of dihydrocapsiate/α-cyclodextrin complex as white powder. According to ¹H-NMR measurement (solvent= DMSO-d6), this complex comprised DCT : α-CD = 1 : 3.8 by molar ratio, and contained 0.44 equivalent by mole of 8-methylnonanoic acid and 1.3 equivalent by mole of ethanol for DCT.
(2) α-Cyclodextrin (486.4 mg) (Tokyo Kasei Kogyo Co., Ltd.; 0.5 mmol) was dissolved in 3 ml of citrate buffer (pH 3.5) at 40°C and then cooled to room temperature to yield a saturated solution of cyclodextrin. Dihydrocapsiate (154.2 mg, 0.5 mmol) was dissolved in 0.5 ml of ethanol at room temperature and added to the saturated solution of cyclodextrin. The solution was stirred at room temperature for 15 minutes, and the precipitated crystals were collected by filtration. The crystals were washed with 5 ml of hexane and dried under reduced pressure to yield 493.2 mg of dihydrocapsiate/α-cyclodextrin complex as white powder. According to ¹H-NMR measurement (solvent= DMSO-d6), this complex comprised DCT : α-CD = 1 : 0.8 by molar ratio, and contained 0.08 equivalent by mole of 8-methylnonanoic acid and 0.2 equivalent by mole of ethanol for DCT.
(3) A dihydrocapsiate/ethanol solution was added to a saturated cyclodextrin solution and stirred for 15 minutes; the mixture was otherwise treated in the same manner as in Example 16 to yield 372.7 mg of dihydrocapsiate/α-cyclodextrin complex as white powder. According to ¹H-NMR measurement (solvent= DMSO-d6), this complex comprised DCT : α-CD = 1 : 1.1 by molar ratio, and contained 0.13 equivalent by mole of 8-methylnonanoic acid and 0.2 equivalent by mole of ethanol for DCT.

Table 7 shows the results of (1) to (3). Thus, the complex can be formed in the mixing ratio of 1 : 1 of the raw materials, DCT : α-CD. As for the solvent for cyclodextrin, both of water and a citrate buffer could be used satisfactorily. When the time required for stirring in the preparation is short, the side chain fatty acid is considered to precede the formation of the complex, and so the stirring is preferably continued for about 1 - 2 hours depending on the condition. When stirred sufficiently, it was confirmed that the complex was formed at approximately 1 : 1 in the ratio of DCT : α-CD.

**Table 7: Formation of α-cyclodextrin complex**

| | Law material | | Solvent | | | Stirring | Yield | Recovery | | Inclusion cmpd. (for DCT) | | |
|---|---|---|---|---|---|---|---|---|---|---|---|---|
| Exmp. | CD | DCT | CD | | DCT (EtOH) | | | Inclusion cmp^{*1} | DCT^{*3} | CD content | Side chain cont^{*3} | Residual EtOH |
| | mmol | mmol | Solv. | ml | ml | hr | mg | [%] | [%] | eq | eq | eq |
| 9-1 | 0.5 | 0.5 | H₂O | 2 | 0.5 | 2 | 493.2 | 77.0 | 90.8 | 0.8 | 0.08 | 0.2 |
| 9-2 | 0.5 | 0.5 | CA* | 3 | 0.5 | 2 | 372.7 | 67.4 | 62.6 | 1.1 | 0.13 | 0.2 |
| 9-3 | 0.5 | 0.5 | CA | 3 | 0.5 | 0.25 | 297.5 | 46.4 | 14.9 | 3.8 | 0.44 | 1.3 |

| | | | | | | | | | | | | |
|---|---|---|---|---|---|---|---|---|---|---|---|---|
| *:aq. citric acid *1:[inclusion compound by wt/(CD+DCT by wt)] x 100 *2:[DCT in inclusion compound by wt/DCT used] x 100 *3: 8-methylnonanoic acid | | | | | | | | | | | | |

### Example 10

### Formation of the complex of α-cyclodextrin and vanillyl decanoate

α-Cyclodextrin (489.3 mg) (Tokyo Kasei Kogyo Co., Ltd.; 0.5 mmol) was dissolved in 2 ml of water at 40°C and then cooled to room temperature to yield a saturated aqueous solution of cyclodextrin. Vanillyl decanoate (155.3 mg, 0.5 mmol) was dissolved in 0.5 ml of ethanol at room temperature and added to the saturated aqueous solution of cyclodextrin'. The solution was stirred at room temperature for 2 hours, and the precipitated crystals were collected by filtration. The crystals were washed with 5 ml of hexane and dried under reduced pressure to yield 546.1 mg of vanillyl decanoate/α-cyclodextrin complex as white powder. According to ¹H-NMR measurement (solvent= DMSO-d6), this complex comprised VD : α-CD = 1 : 1.4 by molar ratio, and contained 0.09 equivalent by mole of n-decanoic acid for VD.

**Table 8: Formation of the complex of vanillyl decanoate and α-cyclodextrin**

| Exmp. | Capsinoid | Yield | Recovery | | Inclusion cmp. (for DCT) | |
|---|---|---|---|---|---|---|
| | | | Inclusion cmp^{*1} | VD^{*2} | CD content | Side chain con^{*3} |
| | | [mg] | [%] | [%] | [eq] | [eq] |
| 10 | vanillyl decanoate | 546.1 | 84.7 | 83.8 | 1.0 | 0.09 |

| | | | | | | |
|---|---|---|---|---|---|---|
| *1:[inclusion compound by wt/(CD+VD by wt)] x 100 *2:[VD in inclusion compound by wt/VD used] x 100 *3: n-decanoic acid | | | | | | |

### Example 11

For the composition comprising γ-cyclodextrin and a triple mixture of capsinoids prepared in Example 1 and for the composition comprising γ-cyclodextrin and dihydrocapsiate prepared in Example 5(2), the solid-state NMR spectra were measured by a 13C-CPMAS technique using an AVANCE 400WB instrument (BRUKER Co., Ltd.).

The results are shown in Fig.. 2. The results of measurement indicated that the triple mixture of capsinoids or dihydrocapsinoid with γ-cyclodextrin exhibited a broader peak (Fig. 2-1, 3) than the simple substance of γ-CD (Fig. 2-2, 4). From these results, it was confirmed that capsinoids and DCT are bound to γ-CD to form the complexes, suggesting the inclusion effect of cyclodextrin to capsinoids, that is, formation of the inclusion compound in the composition of the invention.

### Example 12

### Preparation of a supplement containing a capsinoid CD complex

A complex (20.7 g) of capsinoid and γ-cyclodextrin prepared in the method as described in Example 1 was admixed with 479.3 g of dextrin, and granulated with a stirring granulator. The granules were dried at 70°C with an air drier to yield a granular supplement containing the capsinoid CD complex.

### Example 13

### Preparation of a dietary supplement containing a complex of capsinoid and γ-cyclodextrin

A liquid portion (40 g starch syrup, 60 g corn syrup, 60 g granulated sugar, 2 g citric anhydride, and 25 g distilled water) was weighed and dissolved under warming. To the dissolved liquid portion was added a medium-chain fatty acid triglyceride, and the mixture was stirred. A solid portion (197 g cereal, and 0.11 g complex of capsinoid and γ-cyclodextrin prepared in the method as described in Example 1) was weighed and homogenized under stirring with a mixer. The liquid portion was poured into the solid portion with stirring, and then the whole mixture was poured into a plastic tray and allowed to stand at room temperature. Next day, the mixture was taken out, divided into five portions, placed on a net and dried to give a dietary supplement containing capsinoid.

### Example 14

### Formation of the complex of a naturally extracted capsinoid and γ-cyclodextrin

(1) Natural capsinoid (1.0 g; capsinoid content about 8 wt%/MCT; containing about 0.26 mmol of 3 species of capsinoids) prepared from "CH-19 sweet" by drying, extraction with hexane and molecular distillation after mixing with MCT (medium-chain fatty acid) was admixed with 1.0 ml of ethanol, to which was added a solution of γ-cyclodextrin (339 mg; 0.26 mmol) dissolved in 0.1% citric acid aqueous solution (3.0 ml), and the mixture was stirred at room temperature overnight (about 20 hours). The precipitated crystals were collected by filtration, washed with hexane, and dried under reduced pressure to yield 345 mg of capsinoid/γ-cyclodextrin complex as light yellow powder (yield of capsinoid: about 57.0%). According to ¹H-NMR measurement, this complex comprised capsinoid : γ-CD = 1 : 1.32 by molar ratio, and contained 13.2 wt% component derived from MCT for complex.
(2) γ-Cyclodextrin (339 mg) and 0.1% citric acid aqueous solution (3.0 ml) were separately added to a natural capsinoid/ethanol solution, and the resulting complex crystals were washed as slurry with hexane, and treated otherwise in the same manner as above to yield 315 mg of capsinoid/γ-cyclodextrin complex as light yellow powder (yield of capsinoid: about 53.0%) . According to ¹H-NMR measurement, this complex comprised capsinoid : γ-CD = 1 : 1.47 by molar ratio, and contained 2.4 wt% component derived from MCT for complex.

### Reference Example

### Synthesis of Capsinoids

The capsinoids used in the experiments were synthesized from fatty acid methyl esters or fatty acids according to the methods as described in the document 1 below or an improved method thereof. The followings describe typical examples.

### (1) Synthesis of vanillyl decanoate

The title decanoate was synthesized according to the method as described in the document 1 below.

Methyl decanoate (2.13 ml, 10.5 mmol), vanillyl alcohol (1.62 g, 10.5 mmol), molecular sieves 4 angstrom (10 g) and Novozyme 435 (2.5 g) were added to acetone (50 ml), and the mixture was stirred at room temperature for 2 days. The reaction mixture was filtered through celite and the filtrate was concentrated under reduced pressure. The concentrated residue was purified by chromatography on silica gel to yield 2.25 g of vanillyl decanoate (7.30 mmol; 73.0%) as a colorless oily material.

¹H-NMR (CDCl3, δ) : 0.87 (t, 3H, J=7.1Hz), 1.18-1.30 (m, 12H), 1.55-1.65 (m, 2H), 2.33 (t, 2H, J=7.7Hz), 3.90 (s, 3H), 5.03 (s, 2H), 5.64 (br, 1H), 6.80-6.90 (m, 3H).

### (2) Synthesis of capsiate

Using methyl trans-8-methyl-6-nonenoate synthesized according to the method as described in the document 2 below, the title capsiate was synthesized according to the method as described in the document 1 below.

¹H-NMR (CDCl3, δ) : 0. 95 (d, 6H, J=6. 74Hz), 1.33-1.40 (m, 2H), 1.59-1.67 (m, 2H), 1.94-1.99 (m, 2H), 2.18-2.23 (m, 1H), 2.33 (t, 2H, J=7.52Hz), 3.89 (s, 3H), 5.02 (s, 2H), 5.26-5.39 (m, 2H), 5.63 (br, 1H), 6. 83-6. 90 (m, 3H).

### (3) Synthesis of dihydrocapsiate

Commercially available 8-methylnonanoic acid was converted into the methyl ester, from which the title compound was synthesized according to the method as described in the document 1 below.

¹H-NMR (CDCl3, δ) : 0.86 (d, 6H, J=6.60Hz), 1.12-1.37 (m, 8H), 1.46-1.64 (m, 3H), 2.32 (t, 2H, J=7.56Hz), 3.89 (s, 3H), 5.02 (s, 2H), 5.63 (br, 1H), 6.83-6.90 (m, 3H).

### (4) Synthesis of nordihydrocapsiate

Using a commercially available methyl 7-methyloctanoate, the title compound was synthesized according to the method as described in the document 1 below.

¹H-NMR (CDCl3, δ) : 0.86 (d, 6H, J=5.64Hz), 1.10-1.16 (m, 2H), 1.22-1.32 (m, 4H), 1.42-1.68 (m, 3H), 2.33 (t, 2H, J=7.68Hz), 3.90 (s, 3H), 5.02 (s, 2H), 5.63 (s, 1H), 6.83-6.90 (m, 3H).

### (5) Synthesis of triple mixture of capsinoids

A triple mixture of fatty acid methyl esters was prepared from commercially available capsaicin according to the method as described in the document 1 below. The triple mixture of capsinoids was prepared from this methyl esters according to the method as described in the document 1 below. The abundance ratio of capsiate, dihydrocapsiate and nordihydrocapsiate was confirmed to be 62 : 30 : 7 by HPLC analysis.

### Document 1:

Enzymatic Synthesis of a Capsinoid by the Acylation of Vanillyl Alcohol with Fatty Acid Derivatives Catalyzed by Lipases. Kenji Kobata, Manami Kawaguchi, and Tatsuo watanabe, Biosci. Biotechnol. Biochem., 66 (2), 319-327, 2002

### Document 2

A General and Stereoselective Synthesis of the Capsaicinoids via the Orthoester Claisen Rearrangement. Harumi Koga, Kouhei Goto, Tomiki Takahashi, Masao Hino, Takashi Tokuhashi and Kazuhiko Orito, Tetrahedoron, 52(25), 8451-8470, 1996

### Industrial Applicability

According to the invention, capsinoids so far being in an oily state are stably pulverized and become soluble in water; thus, the capsinoids are provided in forms which can be handled conveniently and used in diverse uses, allowing utilization in the field of a variety, for example pharmaceutical preparations and healthy food products.

### Fig. 1

Residual ratio of capcinoid (%)
Preservation duration (month)
α-CD capcinoid 5°C

## Claims

1. A composition comprising a capsino'id compound and cyclodextrin.

2. A composition as claimed in Claim 1, wherein the capsinoid compound is capsiate, dihydrocapsiate, nordihydrocapsiate or a mixture of two or more species.

3. A composition as claimed in Claim 1 or 2, wherein cyclodextrin is one selected from the group consisting of α-cyclodextrin, β-cyclodextrin, and γ-cyclodextrin.

4. A composition as claimed in any one of Claims 1 to 3, wherein the capsinoid compound is a mixture of capsiate, dihydrocapsiate, and nordihydrocapsiate, and the cyclodextrin is γ-cyclodextrin.

5. A composition as claimed in any one of Claims 1 to 3, wherein the capsinoid compound is dihydrocapsiate, and the cyclodextrin is γ-cyclodextrin.

6. A composition as claimed in any one of Claims 1 to 5, wherein the capsinoid compound and the cyclodextrin form a complex at a molar ratio of 2 : 1 to 1 : 2.

7. A food composition comprising a composition as described in any one of Claims 1 to 6 and a cyclodextrin forming no complex.

8. A process for producing a composition comprising a capsinoid compound and cyclodextrin, which comprises admixing a capsinoid compound with cyclodextrin, followed by drying and pulverizing.

9. A process as claimed in Claim 8, which comprises admixing a capsinoid compound with cyclodextrin in the molar ratio of 4 : 1 to 1 : 10.
